# EUROPÄISCHE PATENTSCHRIFT

(11) **EP 2 845 622 B1**
(45) Veröffentlichungstag und Bekanntmachung des Hinweises auf die Patenterteilung: **21.02.2018**
(21) Anmeldenummer: 13004327.6
(22) Anmeldetag: 04.09.2013
(51) Int. Cl.: A61N 1/04, A61N 1/05, A61N 1/368, A61N 1/39

(54) **Temporär implantierbare Elektrodenanordnung für die Stimulation und intrakardiale Kardioversion des Herzens nach einer Operation**
Temporarily implantable electrode assembly for stimulation and intracardial cardioversion of the heart after surgery
Agencement d'électrodes pouvant être implanté temporairement pour la stimulation et la cardioversion intracardiaque du coeur après une opération

(43) Veröffentlichungstag der Anmeldung: 11.03.2015
(73) Patentinhaber: Peter Osypka Stiftung, 79639 Grenzach-Wyhlen (DE)
(72) Erfinder: Osypka, Peter, 79639 Grenzach-Wyhlen (DE)

(56) Entgegenhaltungen:
- DE-A1-102011 111 649
- DE-U1-202010 011 244
- DE-U1-202010 016 681
- US-A- 5 674 251

## Beschreibung

Die Erfindung betrifft eine temporär implantierbare elastische Elektrodenanordnung mit Fixierungselementen für die Überwachung und / oder Stimulation des Herzens und / oder zur Kardioversion bei Vorhofflimmem nach einer Herzoperation. Die Elektrodenanordnung ist aufgrund ihrer Elastizität leicht auf der Rückseite des Herzens zu positionieren und zu fixieren ohne dass das Epikard verletzt wird. Nach der Anwendung ist die Elektrodenanordnung wieder leicht entfernbar.
Temporäre Myokardiale Elektroden (auch als Herzdrähte bekannt) ermöglichen nach einer Herzoperation eine externe Stimulation des Herzens. Derartige Elektroden sind seit vielen Jahren bekannt und werden routinemäßig nach jeder offenen Herzoperation zur Stimulation der Vorhöfe und Ventrikel eingesetzt. Die Fixierung und damit die Stimulation der Vorhöfe des Herzens, insbesondere die Stimulation des linken Vorhofes mit Herzdrähten, bereitet wegen der speziellen anatomischen Lage des linken Vorhofs erhebliche Schwierigkeiten. Vom linken Vorhof ist bei einer offenen Herz Operation höchstens das linke Herzohr sichtbar, das wegen seiner fragilen Gewebestruktur für eine Fixierung von Herzdrähten nicht geeignet ist. Der eigentliche linke Vorhof befindet sich auf der Rückseite des Herzens und ist für eine temporäre Elektrodenfixierung schwer zugänglich. Daher wird auf die Stimulation des linken Vorhofs, trotz seiner großen medizinischen Bedeutung für die Prophylaxe bzw. Therapie des Vorhofflimmerns, oft verzichtet. DE202010016681 und DE102011111649 offenbaren implantierbare epikardiale Elektrodenanordnungen zur Stimulation und Defibrillation des Herzens. Es besteht daher die Aufgabe eine Elektrodenanordnung zu schaffen, die auf der Herzrückseite um den linken und rechten Vorhof gelegt werden kann, die leicht zu fixieren ist und die in Gebrauchsstellung ihre Position auf den Vorhöfen beibehält. Die Elektrodenanordnung soll es ermöglichen, einfacher und schneller die Vorhöfe des Herzens getrennt oder gemeinsam zu stimulieren bzw. zu kardiovertieren (defibrillieren). Nach beendeter Therapie muss die Elektrodenanordnung ohne mechanische Beeinträchtigung (Reibung) des Epikards wieder leicht zu entfernen sein.
Die Aufgabe wird gelöst durch eine Elektrodenanordnung, die durch eine gezielt abgestimmte Kombination einer elastischen Defibrillationselektrode mit einem elastischen Fixierungselement zur reversiblen Fixierung der Defibrillationselektrode, die für die Therapie unumgängliche physisch stabile Position der Elektroden um den linken Vorhof/die Vorhöfe erlaubt.

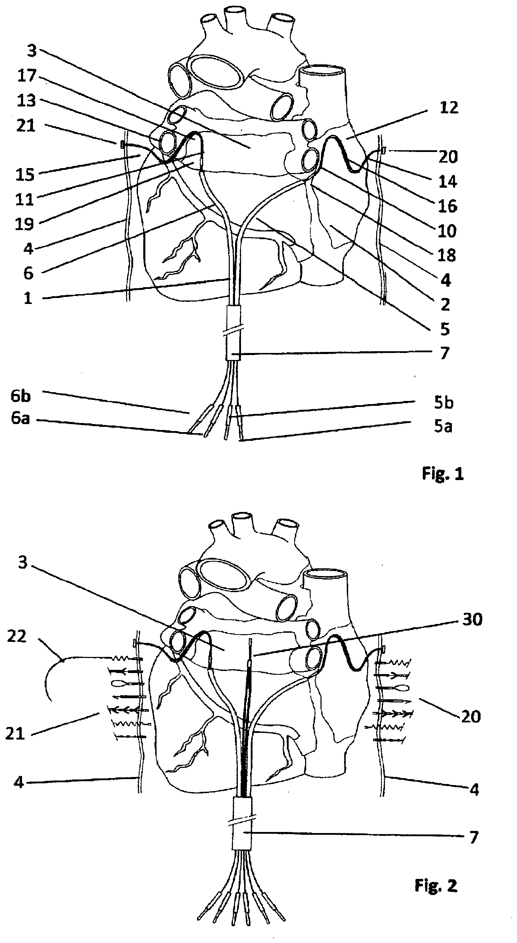

Die Erfindung betrifft somit aus einer Elektrodenanordnung (1) gemäß Anspruch 1. Die Elektrodenanordnung ermöglicht es zwischen den Polpaaren (16), (18) und (17), (19) sowohl zu stimulieren als auch zu kardiovertieren. Außerdem besteht die Möglichkeit, zwischen den indifferenten Polen (16), (17) die Vorhöfe des Herzen gleichzeitig zu kardiovertieren.
Die Schutzschläuche (5), (6) dienen zum Schutz des Epikards. In den Schutzschläuchen (5), (6) verlaufen die elektrischen Zuleitungen zu den Polen der Elektroden. Die Zuleitungen sind zweckmäßigerweise eine flexible Litze.
Falls gewünscht können zusätzliche Stimulationselektroden zur Stimulation des linken und/oder rechten Vorhofs sowie zur Stimulation der Ventrikel vorhanden sein.
Eine zusätzliche bipolare Stimulationselektrode zur Stimulation des linken Vorhofs ist in Fig. 2 dargestellt. Die elektrische Zuleitung für die Stimulationselektrode wird dabei zweckmäßigerweise auch durch den Führungsschlauch (7) geführt.

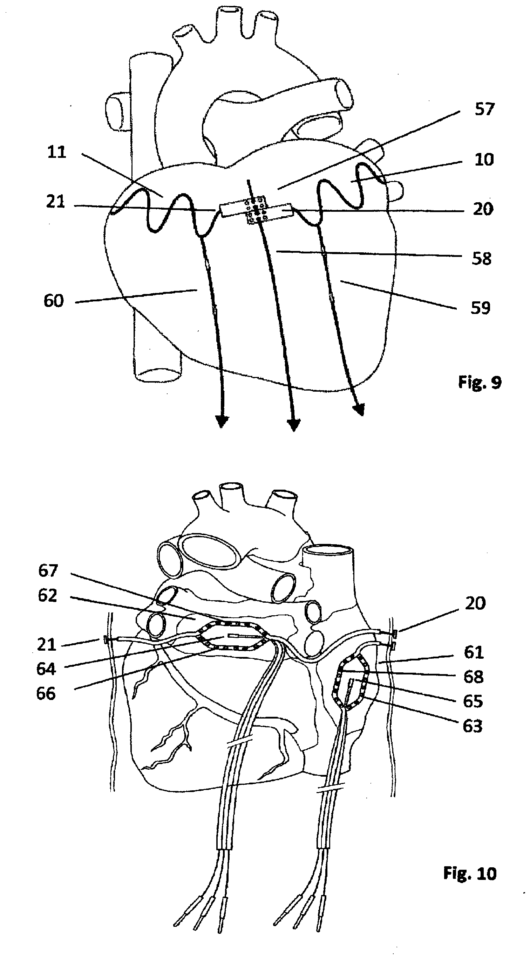

Fig. 9 zeigt Stimulationselektroden für die Ventrikel.
In einer bevorzugten Ausführungsordnung ist der Führungsschlauch vorhanden. (Anspruch 2)

Der Führungsschlauch hat den Vorteil, dass nach erfolgter Fixierung der Defibrillationselektroden durch einfaches Verschieben des Führungsschlauches ein Nachjustieren der Position der Elektrodenpole möglich wird.
Der Führungsschlauch ist aus Kunststoff, beispielsweise aus Polyethylen. Er bildet einen Kanal für die Schutzschläuche (5) und (6) und ist in seiner Längsstreckenrichtung verschiebbar. Am oberen Ende des Führungsschlauches treten die Schutzschläuche (5) und (6) aus. Die Elektrodenpole werden mit den elektrischen Zuleitungen, die in den Schutzschläuchen verlaufen, verbunden. Am unteren Ende werden die Schutzschläuche durch eine Öffnung im Brustkorb nach außen geführt und die elektrischen Zuleitungen mit einem Herzschrittmacher oder Defibrillator verbunden.

Bewegt man den Führungsschlauch nach oben so nähern sich die Schutzschläuche (5), (6) an. Dadurch verkleinert sich der Abstand zwischen den Elektroden-Polen. Wird der Führungsschlauch nach unten gezogen vergrößert sich der Abstand zwischen den Elektroden-Polen. Damit kann jede Elektrodenanordnung den anatomischen Verhältnissen des jeweiligen Herzens leicht angepasst werden.

Das Defibrillationselektrode ist proximal mit der elektrischen Zuleitung verbunden und trägt distal das Fixierungselement.

In einer Ausführungsform besteht die Defibrillationselektrode aus isolierter Litze und einem Poldraht. (Anspruch 3). Der Poldraht stellt den indifferenten Elektrodenpol dar. Der Poldraht besteht aus einem leitfähigen metallischen Material, vorzugweise aus einem Formgedächtnismaterial wie Nitinol. (Anspruch 4)
Der Poldraht kann durch das Aufschieben mindestens einer Wendel verstärkt werden. (Anspruch 5) Es können auch mehrere Wendeln aufgeschoben werden. Die Wendei besteht aus einem elektrisch leitfähigen Material, vorzugsweise Platin oder Edelstahl und verstärkt den Poldraht und verbessert so den Kontakt zur Herzoberfläche.

Der differente Elektrodenpol (Stimulationselektrode) kann auf der Defibrillationselektrode angebracht werden.
Der differente Elektrodenpol ist beispielsweise als gegenüber dem Poldraht Isolierte Klemmhülse ausgebildet, die auf dem Poldraht sitzt oder besteht ebenfalls aus einem Teilstück des Poldrahts. (Anspruch 9)

Der Poldraht kann auf der dem Herzen abgewandten Seite durch eine Beschichtung mit Kunststoff isoliert werden. Durch diese Maßnahme wird ein Stromfluss in die schmerzempfindlichen Herz-abgewandten Seiten vermieden.

Der Defibrillationspol kann auch aus einer Edelstahllitze bestehen. (Anspruch 6)
Der differente Elektrodenpol ist beispielsweise als Klemmhülse ausgebildet, die auf der Litze sitzt oder besteht ebenfalls aus einem Teilstück der Litze.
Die Litze kann auf der dem Herzen abgewandten Seite durch eine Beschichtung mit Kunststoff isoliert werden.

In einer Ausführungsform ist die Oberfläche der metallischen Pole mit einer Schicht aus Platin oder Gold beschichtet. (Anspruch 7)
Die Beschichtung der Elektrodenpole mit einer Edelmetallschicht ist besonders wichtig, um bei einer Folge von Kardioversionen die Bildung einer Oxydschicht zu verhindern.

In einer Ausführungsform ist die Defibrillationselektrode in Schlauchform ausgebildet. (Anspruch 8)
Der Schlauch besteht aus einem flexiblen Material. Geeignetes Material ist z.B. Silikon, Dacron, Polyurethan, Polyester, Seide oder Polyimid. Der Schlauch besitzt mindestens ein Lumen zur Aufnahme der Elektroden. Auf der dem Herzen zugewandten Seite besitzt der Schlauch mehrere Öffnungen für den indifferenten Pol und eine Öffnung für den differenten Pol die bei der Stimulation/ Kardioversion einen elektrischen Stromfluss ermöglichen. Durch diese Maßnahme wird ein Stromfluss in die schmerzempfindlichen Herzabgewandten Seiten vermieden.
In dieser Ausführungsform in Schlauchform bestehen die beiden Pole aus einer Wendel aus beispielweise sechs isolierten Drähten aus Edelstahl, die in dem Schlauch mit entsprechenden Öffnungen integriert ist. Innerhalb der Wendel befindet sich eine niederohmige Zuleitung, beispielsweise eine Zuleitung aus Silber oder einem Edelmetall, die distal mit der Wendel elektrisch verbunden ist. An den entsprechenden Öffnungen des Schlauches wird die isolation von vier Wendeldrähten entfernt, sodass bei Bedarf ein elektrischer Strom durch die parallel angeordneten Drähte fließen kann.
Die beiden verbleibenden zwei isolierten Drähte der Wendel bilden proximal, nach Entfernen der Isolation an der dafür vorgesehenen Öffnung, den differenten Elektrodenpol. Durch Auftragen von Edelmetall mit Hilfe der LASER Technik auf den Polen in den Öffnungen (Bumps), können die elektrischen Eigenschaften der Pole, bezüglich der Oxydschicht Bildung, verbessert werden.

### Elastizitätsabschnitt:

Wesentlich ist die Elastizität der Defibrillationselektrode, die durch einen sogenannten Elastizitätsabschnitt bedingt wird. Elastizitätsabschnitt bezeichnet einen Bereich auf der Defibrillationselektrode der sich auseinanderziehen lässt und der nach erfolgter Fixierung der Defibrillationselektrode sich wieder in seine ursprüngliche Form zusammenzieht Dies lässt sich beispielsweise erreichen durch Formung der Defibrillationselektrode in Wellenform, Kreisform, Ellipsenform, als Polygon (Hexagon) oder jede andere Form, die sich auseinanderziehen lässt. Besonders geeignetes Material zur Formung ist Formgedächtnismaterial wie Nitinol. In obigem Beispiel ist die Welle, der Kreis, die Ellipse, das Hexagon der Elastizitätsabschnitt. (Anspruch 10)
Es ist aber auch denkbar, dass die Defibrillationselektrode oder Teile davon aus einem dehnbaren Kunststoff wie z.B. Silikon besteht auf dem die Elektrodenpole angebracht werden.
Der Elastizitätsabschnitt ermöglicht, dass die Defibrillationselektrode beim Platzieren der Elektrodenanordnung um die Vorhöfe auf der rückwärtigen Seite des Herzens auseinandergezogen werden kann, was das Hochschieben der Elektrodenanordnung unter das Herz erleichtert. Der Elastizitätsabschnitt ermöglicht gleichzeitig, dass die Fixierungselemente durch das Perikard gezogen werden können. Nachdem die Fixierungselemente positioniert sind, nimmt die Defibrillationselektrode ihre ursprüngliche Form wieder an und sorgt so für einen optimalen stabilen Kontakt der Elektrodenpole auf der Herzoberfläche. Beim Zusammenziehen der Defibrillationselektrode in die ursprüngliche Form wird gleichzeitig die Verankerung im Perikard erreicht.
Ferner ermöglicht der Elastizitätsabschnitt eine Flexibilität in der Konstruktion und insbesondere in der Formgebung, die es erlaubt, dass Bypässe oder andere durch die Operation entstandenen Wunden umgangen werden können.

### Fixierungselement:

Zur Fixierung trägt die Defibrillationselektrode am distalen, dem Perikard zugewandten Ende ein Fixierungselement. Das Fixierungselement muss zwei Funktionen erfüllen. Zum einen muss eine stabile Verankerung während der Dauer der Behandlung gegeben sein, andererseits muss sich die Verankerung nach erfolgter Behandlung auch leicht wieder lösen, sodass ein späteres Entfernen durch eine kleine Öffnung bei sonst geschlossenem Thorax möglich wird.

Als Fixierungselemente zur Fixierung im Perikard eignen sich alle aus dem Stand der Technik bekannten Elemente wie beispielsweise Zig-Zag, Anker, Ösen, Loop, Wendel, V-Form, elastischer T-bar, Knoten. (Anspruch 11)
Die Fixierungselemente (20) und (21) können gleich oder verschieden sein. In einer bevorzugten Ausführungsform sind sie gleich, das heißt sowohl Fixierungselement (20) als auch Fixierungselement (21) sind beispielsweise ein Anker.

Das Fixierungselement wird beispielsweise mit einer Herznadet durch das Perikard gezogen. Nach Fixierung wird die Herznadel abgeschnitten.
Die Positionierung und Fixierung kann auch mit Hilfe eines Einführungsbestecks erfolgen.

Da die anatomische Größe der Herzen sehr unterschiedlich sein kann, ist der Abstand zwischen dem Perikard und dem Beginn der Vorhöfe wichtig. Man erreicht den richtigen Abstand, indem man die Fixierungselemente (z.B. Anker oder Zig-Zag) so lang konstruiert, dass sie bei jeder gewünschten Lage der Elektrodenpole aktiviert werden können.

In einer Ausführungsform ist das Fixierungselement beispielsweise eine Anzahl von Ankern. Die Verankerung kommt durch das Aufspleißen des Ankers zustande. Mit der Herznadel wird die Defibrillationselektrode durch das Perikard gezogen bis der gewünschte Anker ausgetreten ist. Durch Zurückziehen der Defibrillationselektrode spleißt der Anker auf. Der Rest wird abgeschnitten.

In einer Ausführungsform ist die Fixierung auf der vorderen Herzseite vorgesehen.
Die beiden Defibrillationselektroden werden rund um die Vorhöfe des Herzens gezogen und die beiden Fixierungselemente werden an der vorderen Herzseite miteinander temporär verbunden. In dieser Ausführungsform bestehen die Fixierungselemente geeigneter Weise aus zwei ineinander greifenden Platten, die zusammengesteckt und mittels eines Fadens zusammengehalten werden. Der Faden wird wie die elektrischen Zuleitungen der Elektrodenanordnung durch eine kleine Körperöffnung nach Außen geführt. Nach Gebrauch kann der Faden gezogen werden und die Fixierungselemente trennen sich, sodass die Elektrodenanordnung von der hinteren Herzseite aus durch die vordere Körperöffnung ebenfalls entfernt werden kann.

Konstruktiv ist es günstig wenn die Abschnitte im vorderen Herzbereich isoliert sind und ebenfalls in Wellenabschnitte geformt sind. Wie oben dargelegt wird durch die elastischen Eigenschaften der Defibrillationselektrode ein fester Sitz der Elektrodenanordnung am Herz erreicht.

Die erfindungsgemässe Elektrodenanordnung erlaubt nicht nur die optimale Kardioversion und Stimulation der Vorhöfe des Herzens, sondern darüber hinaus auch eine gleichzeitige Stimulation der Ventrikel. Hierzu werden an geeigneter Stelle der Elektrodenanordnung im vorderen oder hinteren Herzbereich bipolare Stimulationsabgänge angebracht.

Der große Vorteil der efindungsgemäßen Elektrodenanordnung besteht darin, dass durch die Konstruktion der Elektrodenanordnung dem Operateur ermöglicht wird zunächst die Defibrillationselektroden am linken und rechten Vorhof zu positionieren, zu fixieren und nach erfolgter Fixierung gegebenenfalls durch einfaches Verschieben des Führungsschlauches ein Nachjustieren vorzunehmen. Somit wird erreicht dass der Elektrodenkontakt zur Herzoberfläche ausgezeichnet ist ohne dass das Epikard verletzt wird. Der Elektrodenkontakt ist so optimiert, dass die Defibrillation mit äußert geringen Schockenergien vorgenommen werden kann. Eine äußert geringe Schockenergie liegt im Bereich von 0,1 Joule und weniger.

### Zeichnungen

Weitere Einzelheiten, Merkmale und Vorteile der Erfindung lassen sich dem folgenden Beschreibungsteil entnehmen, in dem anhand der Zeichnungen und Ausführungsbeispielen die Erfindung näher erläutert wird. Sie zeigen in schematischer Darstellung in
**Fig.1** Eine beispielhafte Elektrodenanordnung mit wellenförmigen Elastizitätsabschnitt über den linken und rechten Vorhof auf der Rückseite des Herzens gelegt und im Perikard verankert.
**Fig.2** Eine beispielhafte Elektrodenanordnung mit wellenförmigen Elastizitätsabschnitt über den linken und rechten Vorhof auf der Rückseite des Herzens gelegt und im Perikard verankert. Mögliche Fixierungselemente werden gezeigt Eine zusätzliche Stimulationselektrode für den linken Vorhof ist vorhanden.
**Fig.3** Eine beispielhafte Elektrodenanordnung mit hexagonalem Elastizitätsabschnitt über den linken und rechten Vorhof auf der Rückseite des Herzens gelegt und im Perikard verankert.
**Fig.4** Eine beispielhafte Elektrodenanordnung mit kreisförmigen Elastizitätsabschnitt über den linken und rechten Vorhof auf der Rückseite des Herzens gelegt und im Perikard verankert.
**Fig.5** Eine beispielhafte Elektrodenanordnung mit einem die beiden Defibrillationselektroden verbindenden Elastizitätsabschnitt.
**Fig.6** Eine beispielhafte Elektrodenanordnung mit wellenförmigen Elastizitätsabschnitt und einer Defibrillationsetektrode in Schlauchform.
**Fig.7** Eine beispielhafte Elektrodenanordnung mit wellenförmigen Elastizitätsabschnitt und einer Defibrillationselektrode in Schlauchform. Die Schlauchabschnitte sind verzweigt.
**Fig. 8** Eine beispielhafte Etektrodenanordnung mit wellenförmigen Elastizitätsabschnitt und einer Defibrillationselektrode in Schlauchform zur Stimulation/Kardioversion des linken Vorhofs.
**Fig.9** Eine beispielhafte Elektrodenanordnung mit wellenförmigen Elastizitätsabschnitt. Die Fixierungselemente sind auf der vorderen Herzseite verankert. Zusätzliche Stimulationselektroden zur Stimulierung der Ventrikel sind vorhanden.
**Fig. 10** Eine beispielhafte Elektrodenanordnung mit getrennten hexagonalen Elastizitätsabschnitten über den linken und rechten Vorhof auf der Rückseite des Herzens gelegen und im Perikard verankert.

### Beschreibung der Abbildungen

Fig. 1 zeigt den hinteren Herzbereich mit einer Fixierung der Elektrodenanordnung im Perikard. Die Elektrodenanordnung (1) besteht aus zwei Defibrillationselektroden (10) und (11). Die indifferenten Pole (16) und (17) sind Poldrähte. Defibrillationselektrode (10) liegt über dem rechten Vorhof (2). Defibrillationselektrode (11) liegt über dem linken Vorhof (3). Die Poldrähte bestehen aus Nitinoldraht der durch Tempern wellenförmig geformt wurde. sodass die Poldrähte je einen Elastizitätsabschnitt (12) bzw. (13) aufweisen. Die Welle bestehend aus Wellenberg und Wellental stellt den Elastizitätsabschnitt dar. Jeder Poldraht mündet in eine elektrisch isolierte Edelstahl Litze (14) bzw. (15), die distal zum Fixierungselement (20) bzw. (21) führt. Das Fixierungselement ist ein elastischer T-Bar beispielsweise aus Silikon, der im Perikard (4) verankert ist. Die Poldrähte (16) bzw. (17) sind durch aufgeschobene Wendeln verstärkt.

Die beiden gegeneinander isolierten Zuleitungen (5a) und (5b) bzw. (6a) und (6b) werden gemeinsam in einem Schutzschlauch (5) bzw. (6) zu dem differenten Elektrodenpol (18) bzw. (19) geführt, der als Klemmhülse ausgebildet ist. Zuleitung (5a) ist mit der Innenseite der Klemmhülse (18) elektrisch leitend verbunden. Zuleitung (5b) führt zu dem indifferenten Poldraht (10). Zuleitung (6a) ist mit der Innenseite der Klemmhülse (19) elektrisch leitend verbunden. Zuleitung (6b) führt zu dem indifferenten Poldraht (11).

Schutzschläuche (5) und (6) werden gemeinsam in dem Führungsschlauch (7) geführt. Der Führungsschlauch (7) ist in Bezug auf die Längsachse verschiebbar, sodass der Abstand zwischen den beiden Defibrillationselektroden (10) und (11) und zwischen den beiden differenten Elektrodenpolen (18) bzw. (19) veränderbar ist. Wird der Führungsschlauch (7) nach oben geschoben so nähern sich die beiden Klemmhülsen (18) und (19) an. Auch die Form und der Abstand der Defibrillationselektroden (10) und (11) verändert sich.

**Fig. 2** zeigt die in Fig. 1 dargestellte Elektrodenanordnung. Hier wird verdeutlicht, dass Fixierungselement (20) bzw. (21) verschiedene Formen annehmen kann, wie z.B. Zig-Zag, Anker, Loop und Haken, Knoten, Mehrfachanker, Mehrfachknoten. Mit der Herznadel (22) wird der Poldraht durch das Perikard (4) gezogen bis das Fixierungselement ausgetreten ist; ist das Fixierungselement ein Mehrfachanker bis der gewünschte Anker ausgetreten ist Durch Zurückziehen des Poldrahts verankert sich das Fixierungselement im Perikard.
Im Ausführungsbeispiel gemäß Fig. 2 besitzt die Elektrodenanordnung eine zusätzliche bipolare Stimulationselektrode (30), deren Zuleitungen ebenfalls durch den Führungsschlauch (7) geführt werden. Die Stimulationselektrode (30) ermöglicht eine weitere Stimulation des linken Vorhofs (3).

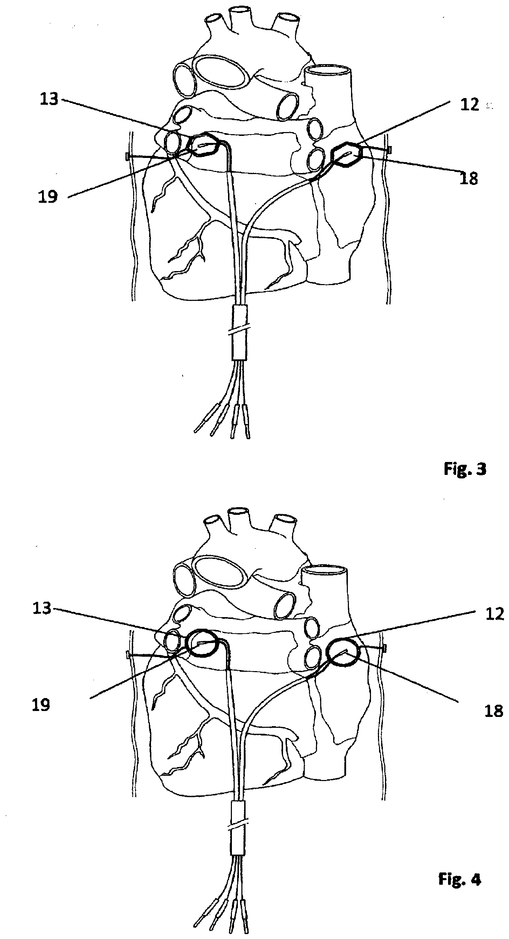

**Fig. 3** zeigt die in Fig. 1 dargestellte Elektrodenanordnung wobei Elastizitätsabschnitt (12) bzw. (13) eine hexagonale Form aufweisen. Die Poldrähte der indifferenten Pole sind im Bereich des Hexagons durch Wendeln verstärkt. Der differente Elektrodenpol (18) bzw. (19) liegen innerhalb des Hexagons.

**Fig. 4** zeigt die in Fig. 1 dargestellte Elektrodenanordnung wobei Elastizitätsabschnitt (12) bzw. (13) eine kreisförmige Form aufweisen. Die Poldrähte der indifferenten Pole sind im Bereich des Kreises durch Wendeln verstärkt. Der differente Elektrodenpol (18) bzw. (19) liegen innerhalb des Kreises.

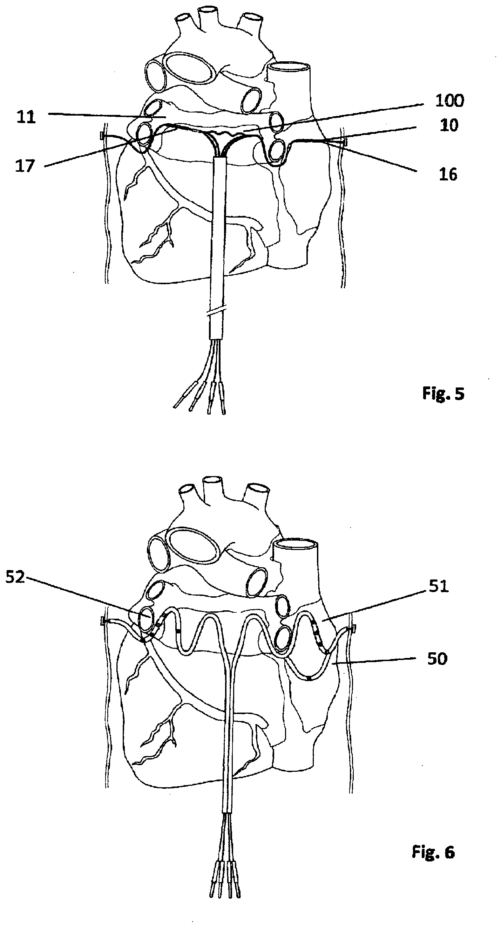

**Fig. 5** zeigt die in Fig. 1 dargestellte Elektrodenanordnung, die sich durch eine spezielle Anordnung des Elastizitätsabschnitts auszeichnet. Die Defibrillationselektroden (10) bzw. (11) mit aufgeschobenen Wendeln (16) bzw. (17) werden durch den Elastizitätsabschnitt (100) verbunden. Der Elastizitätsabschnitt besteht beispielsweise aus wellenförmig geformtem isoliertem Faden oder aus einem dehnbaren Silikonfaden, der sowohl an Defibrillationselektrode (10) als auch an Defibrillationselektrode (11) befestigt ist.

**Fig. 6** zeigt die in Fig. 1 dargestellte Elektrodenanordnung, die sich durch eine spezielle wellenförmige Anordnung der Defibrillationselektrode in Schutzschlauchform über dem linken und rechten Vorhof auf der Rückseite des Herzens auszeichnet. Über dem rechten Vorhof befinden sich zwei Schutzschlauchabschnitte (50) mit mehreren Öffnungen für den indifferenten Pol (51) und mit einer Öffnung für den differenten Pol. Die beiden Schutzschlauchabschnitte sind so geformt, dass die Öffnungen beim Positionieren der Elektrodenanordnung stets zur Herzseite zeigen und bei der Abgabe eines Schockimpulses ein Stromfluss in die schmerzempfindlichen Herzabgewandten Seiten vermieden wird. Die Öffnungen des wellenförmigen Schutzschlauchabschnittes (52) über dem linken Vorhof erfüllen den gleichen Zweck.

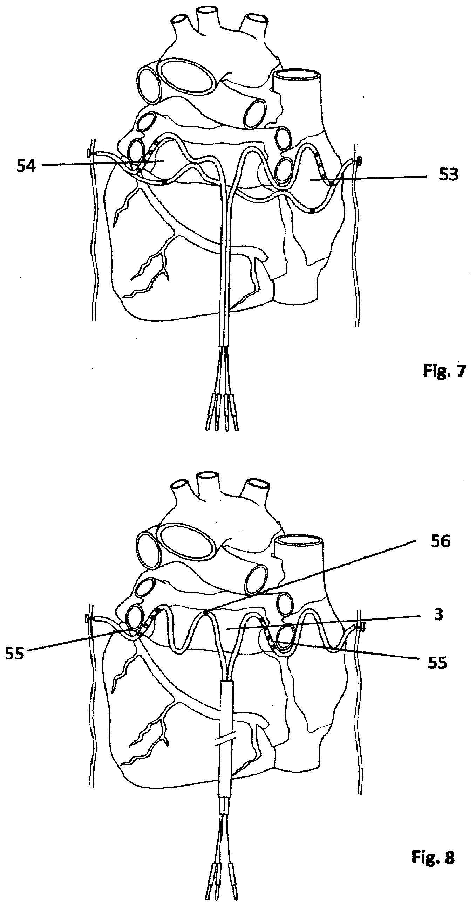

**Fig.7** zeigt eine weitere beispielhafte Elektrodenanordnung mit wellenförmigen Elastizitätsabschnitt und einer Defibrillationselektrode in Schutzschlauchform über dem linken und rechten Vorhof auf der Rückseite des Herzens. Hier sind die Schutzschlauchabschnitte (53), (54) so geformt, dass ein unbeabsichtigtes Verdrehen der Öffnungen während der Positionierung verhindert wird.

**Fig.8** zeigt eine weitere beispielhafte Elektrodenanordnung mit wellenförmigen Elastizitätsabschnitt, in der nur der linke Vorhof (3) kardiovertiert / stimuliert werden kann. Der rechte Vorhof kann durch eine gesonderte Elektrodenanordnung auf der Rückseite des Herzens aber auch auf der Vorderseite des Herzens kardiovertiert / stimuliert werden. Die elastische Schutzschlauchförmige Elektrodenanordnung über dem linken Vorhof weist zwei separate indifferente Pole und einen differenten Pol (55) auf. Zwischen dem differenten Pol (56) und den indifferenten Polen kann der linke Vorhof stimuliert und zwischen den beiden indifferenten Polen kardiovertiert werden. Damit kann die notwendige Schockenergie für die Kardioversion auf ein Minimum reduziert werden, was für den Patienten bezüglich der Schmerzempfindung von Vorteil ist,

**Fig. 9** zeigt die Vorderseite des Herzens. Die Defibrillationselektroden (10) und (11) tragen die Fixierungselemente (20) und (21), die aus zwei ineinander greifenden Platten (57), die zusammengesteckt und mittels eines Fadens (58) zusammengehalten werden. Nach Gebrauch kann der Faden gezogen werden und die Fixierungselemente trennen sich, sodass die Elektrodenanordnung von der hinteren Herzseite aus durch die vordere Körperöffnung ebenfalls entfernt werden kann. Die Defibrillationselektroden (10) und (11) sind wellenförmig über den vorderen Herzbereich bis zu den ineinander greifenden Platten weiter geführt. Im vorderen Herzbereich über den Ventrikeln sind zusätzliche separate bipolare Elektroden (59), (60) angebracht, die zur Stimulierung des rechten und linken Ventrikels dienen.

**Fig. 10** zeigt eine weitere Ausführungsform der erfindungsgemässen Elektrodenanordnung, wobei die Elastizitätsabschnitte eine hexagonale Form (61), (62) aufweisen. Die den ganzen linken Vorhof überdeckende hexagonale indifferente Elektrode (62) ist separat an den beiden Seiten des Perikards fixiert (20), (21). Die über dem rechten Vorhof platzierte hexagonale indifferente Elektrode (61) wird an einer Seite des Perikards so platziert, dass die Platzierung der besonderen anatomischen Form des rechten Vorhofs gerecht wird. Die Stimulation erfolgt jeweils zwischen den differenten Polen (63), (64) innerhalb des Hexagons und dem länglichen Außenabschnitten (65), (66) des Hexagons. Die Kardioversion erfolgt jeweils separat im linken und rechten Vorhof. Im linken Vorhof erfolgt die Kardioversion zwischen den elektrisch separaten Polschenkeln des oberen (67) und des unteren (66) Teils des Hexagons. Im rechten Vorhof erfolgt die Kardioversion zwischen den elektrisch separaten Polen des rechten (65) und des linken (68) Polschenkels des Hexagons.

## Patentansprüche

1. Elektrodenanordnung (1) zur temporären Kardioversion und/oder zur temporären Stimulation des Herzens nach einer Operation am offenen Herzen bestehend aus:
a) einer ersten Defibrillationselektrode (11) mit mindestens einem indifferenten Pol (17) und einem Elastizitätsabschnitt (13), wobei die Defibrillationselektrode (11) in Gebrauchsstellung über dem linken Vorhof (3) gelegt werden kann, distal das Fixierungselement (21) aufweist und proximal in einen Schutzschlauch (6) mündet;
b) aus einer zweiten Defibrillationselektrode (10) mit mindestens einem indifferenten Pol (16) und einem Elastizitätsabschnitt (12), wobei die Defibrillationselektrode (10) in Gebrauchsstellung über dem rechten Vorhof (2) gelegt werden kann, distal das Fixierungselement (20) aufweist und proximal in einen Schutzschlauch (5) mündet;
c) aus den differenten Polen (18),(19);
wobei die Fixierungselemente (20) und (21) in Gebrauchsstellung rechts- bzw. linksseitig im Perikard (4) fixiert werden können oder an der Vorderseite des Herzens miteinander verankert werden können, und wobei die Schutzschläuche (5) und (6), in denen die elektrischen Zuleitungen zu den Polen der Elektroden verlaufen und gemeinsam in einem Führungsschlauch (7) geführt werden, wobei der Führungsschlauch in Bezug auf die Längsachse verschiebbar ist.

2. Elektrodenanordnung gemäß Anspruch 1 wobei die indifferenten Pole (16) und (17) Poldrähte sind.

3. Elektrodenanordnung gemäß Anspruch 2, wobei der Poldraht aus Nitinol besteht.

4. Elektrodenanordnung gemäß Anspruch 2 oder 3, wobei der Poldraht durch mindestens eine Wendel verstärkt ist.

5. Elektrodenanordnung gemäß Anspruch 1 wobei die indifferenten Pole (16) und (17) aus Litze geformt sind.

6. Elektrodenanordnung gemäß einem der Ansprüche 1-5, wobei die Oberfläche der metallischen Pole (16) und (17) mit einer Schicht aus Platin oder Gold beschichtet ist.

7. Elektrodenanordnung gemäß Anspruch 1, wobei die Defibrillationselektrode ein Schlauch ist mit mindestens einem Lumen zur Aufnahme der Elektroden und mit mehreren Öffnungen für den indifferenten Pol und einer Öffnung für den differenten Pol.

8. Elektrodenanordnung gemäß einem der Ansprüch 1-7, wobei die differenten Pole (18), (19) als Klemmhülse ausgebildet sind.

9. Elektrodenanordnung gemäß einem der Ansprüche 1-8, wobei der Elastizitätsabschnitt in Wellenform, Kreisform, Ellipsenform oder als Polygon (Hexagon) vorliegt.

10. Elektrodenanordnung gemäß einem der Ansprüche 1-9, wobei das Fixierungselement ein Zig-Zag-Element, ein Anker, eine Öse, ein Loop, eine Wendel, ein V-förmiges Element, ein elastischer T-bar, oder ein Knoten ist.

## Claims

1. Electrode assembly (1) for temporary cardioversion and/or temporary stimulation of the heart after open heart surgery comprising:
a) a first defibrillation electrode (11) with at least one indifferent pole (17) and with an elastic section (13), whereby the defibrillation electrode (11) is configured to be positioned over the left atrium (3) in the use position, said defibrillation electrode comprises a fixation member (21) disposed on its distal end and is proximally guided into a protective tube (6);
b) a second defibrillation electrode (10) with at least one indifferent pole (16) and with an elastic section (12), whereby the defibrillation electrode (10) is configured to be positioned over the right atrium (2) in the use position, said defibrillation electrode comprises a fixation member (20) disposed on its distal end and is proximally guided into a protective tube (5); and
c) a pair of different poles (18), (19);
whereby the fixation members (20) and (21) are configured to be fixed in the use position on the right and left side within the pericardium or are configured to be anchored to each other at the front of the heart; and
whereby the electrical supply cables to the poles are inside the protective tubes (5) and (6) which are both guided into a guide tube (7) which can be slid along its longitudinal axis.

2. The electrode assembly according to claim 1 wherein the indifferent poles (16) and (17) are pole wires.

3. The electrode assembly according to claim 2, wherein the pole wires are made of nitinol.

4. The electrode assembly according to claim 2 or 3, wherein the pole wires are reinforced by at least one coil.

5. The electrode assembly according to claim 1 wherein the indifferent poles (16) and (17) are formed from stranded wire.

6. The electrode assembly according to any one of claims 1 to 5, wherein the surface of the metallic poles (16) and (17) is coated by a layer of platinum or gold.

7. The electrode assembly according to claims 1 , wherein the defibrillation electrode is a tube with at least one lumen to receive the electrodes and with several openings for the indifferent pole and one opening for the different pole.

8. The electrode assembly according to any one of claims 1 to 7, wherein the different poles (18), (19) are configured as clamping sleeves.

9. The electrode assembly according to any one of claims 1 to 8, wherein the elastic section is present in wave form, circular form, elliptical form or a polygon (hexagon).

10. The electrode assembly according to any one of claims 1 to 9, wherein the fixing member is selected from the group consisting of a zigzag element, an anchor, an eyelet, a loop, coil, a V-shaped element, an elastic T-bar, or a knot.

## Revendications

1. Ensemble d'électrodes (1) pour la cardioversion temporaire et / ou pour la stimulation temporaire du coeur après une opération à coeur ouvert, comprenant:
a) une première électrode de défibrillation (11) avec au moins un pôle indifférent (17) et une partie élastique (13), l'électrode de défibrillation (11) se trouve dans la position d'utilisation au-dessus de l'oreillette gauche (3), l'électrode de défibrillation comprend une élément de fixation (21) à l'extrémité distale et s'étend à l'extrémité proximale dans un tuyau de protection (6);
b) une deuxième électrode de défibrillation (10) avec au moins un pôle indifférent (16) et une partie élastique (12), l'électrode de défibrillation (10) se trouve dans la position d'utilisation au-dessus de l'oreillette droite (2), l'électrode de défibrillation comprend une élément de fixation (20) à l'extrémité distale et s'étend à l'extrémité proximale dans un tuyau de protection (5);
c) des pôles differents (18), (19);
dans la position d'utilisation les éléments de fixation (20) et (21) peuvent être fixés dans le péricarde (4) sur le côté droit ou sur le côté gauche ou peuvent être ancrés les uns aux autres à l'avant du coeur,
les conduits électriques aux pôles passent à l'intérieur des tuyaux de protection (5) et (6) lesquelles s' etendent à travers un tube de guidage (7); le tube de guidage peut être déplacé par rapport à son axe longitudinal.

2. Ensemble d'électrodes selon la revendication 1, dans lequel les pôles indifférents (16) et (17) sont formés à partir d'un fil.

3. Ensemble d'électrodes selon la revendication 2 , dans lequel les pôles sont formés à partir des fils de nitinol.

4. Ensemble d'électrodes selon la revendication 2 ou 3, dans lequel les fils sont renforcés par au moins une hélice.

5. Ensemble d'électrodes selon 1, dans lequel les pôles indifférents (16) et (17) sont formés à partir du fil torsadé.

6. Ensemble d'électrodes selon l'une quelconque des revendications 1-5, dans lequel la surface des pôles métalliques (16) et (17) est revêtue d'une couche de platine ou d'or.

7. Ensemble d'électrodes selon la revendication 1, dans lequel l'électrode de défibrillation est formé d'un tube avec au moins une lumière destinée à recevoir l'électrode et ayant une pluralité d'ouvertures pour le pôle indifférent et une ouverture pour l'autre pôle.

8. Ensemble d'électrodes selon l'une quelconque des revendications 1-7, dans lequel les pôles différents (18), (18) sont réalisés sous la forme d'une douille de serrage.

9. Ensemble d'électrodes selon l'une quelconque des revendications 1-8, dans lequel la partie élastique est de forme ondulée, de forme circulaire, de forme elliptique, ou de forme d'un polygone (hexagone).

10. Ensemble d'électrodes selon l'une quelconque des revendications 1-9, dans lequel l'élément de fixation est un élément de zig-zag, une ancre, un oeillet, une boucle, une hélice, un élément en forme de V, une barre en T élastique ou un noeud.
